(19) Europäisches Patentamt
European Patent Office
Office européen des brevets

(11) Veröffentlichungsnummer: **0 032 167**
**A1**

(12) **EUROPÄISCHE PATENTANMELDUNG**

(21) Anmeldenummer: **80106894.1**

(22) Anmeldetag: **08.11.80**

(51) Int. Cl.³: **C 07 C 15/44**
C 08 F 2/38, C 08 F 4/70
C 08 F 36/04

(30) Priorität: **10.01.80 DE 3000708**

(43) Veröffentlichungstag der Anmeldung:
**22.07.81 Patentblatt 81/29**

(84) Benannte Vertragsstaaten:
**AT BE FR GB IT NL**

(71) Anmelder: **CHEMISCHE WERKE HÜLS AG**
**Postfach 1320**
**D-4370 Marl 1(DE)**

(72) Erfinder: **Hahn, Karl, Dr.**
**Am Schlagbaum 15**
**D-4370 Marl(DE)**

(72) Erfinder: **Kampf, Wolfgang, Dr.**
**Im Wienäckern 60 b**
**D-4358 Haltern(DE)**

(54) **Verfahren zur Herstellung von Reaktionsprodukten aus konjugierten Diolefinen und aromatischen Kohlenwasserstoffen.**

(57) Herstellung von Reaktionsprodukten aus konjugierten Diolefinen und aromatischen Kohlenwasserstoffen mittels eines Katalysators bestehend aus
1 einer phosphormodifizierte Kobaltverbindung,
2 einer halogenhaltigen aluminiumorganischen Verbindung und
3 einer Verbindung
der allgemeinen Formeln,

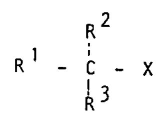
1,

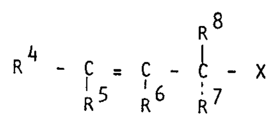
11 oder

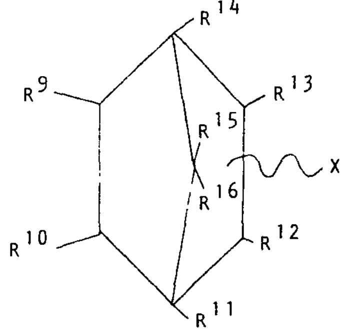
111

./...

EP 0 032 167 A1

wobei

X jeweils Chlor, Brom oder Jod und $R^1$ bis $R^{16}$ unabhängig voneinander gegebenenfalls durch Halogenatome substituierte (cyclo)-aliphatische Kohlenwasserstoffreste mit 1 bis 20 Kohlenstoffatomen oder gegebenenfalls durch 1 bis 5- gegebenenfalls durch Halogenatome substituierte - (cyclo) - aliphatische Kohlenwasserstoffreste mit 1 bis 10 Kohlenstoffatomen und/oder durch Halogenatome substituierte Arylreste mit 6 bis 14 Kohlenstoffatomen bedeuten, $R^2$ bis $R^{16}$ auch Wasserstoff sein können, jedoch $R^2$ und $R^3$ nur dann, wenn $R^1$ einen gegebenenfalls durch Halogenatome substituierten Arylrest mit 6 bis 14 Kohlenstoffatomen darstellt, und $R^9$ und $R^{10}$ zusammen auch Bestandteile eines anellierten - gegebenenfalls substituierten - Ringsystems sein können.

## Verfahren zur Herstellung von Reaktionsprodukten aus konjugierten Diolefinen und aromatischen Kohlenwasserstoffen

Die Alkenylierung aromatischer Kohlenwasserstoffe durch Diene ist in der Literatur mehrfach beschrieben worden (u. a. G. A. Olah, Friedel-Crafts and Related Reactions, Vol. II/1). Ihre Darstellung erfolgt mit Hilfe homogener und heterogener saurer Katalysatoren, die vorwiegend zu Alkenyl- und/oder Dialkenylderivaten aromatischer Kohlenwasserstoffe der allgemeinen Formeln

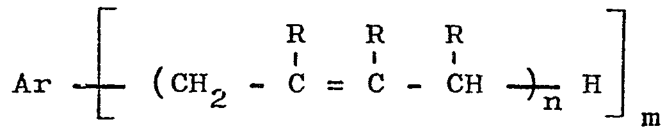 und/oder

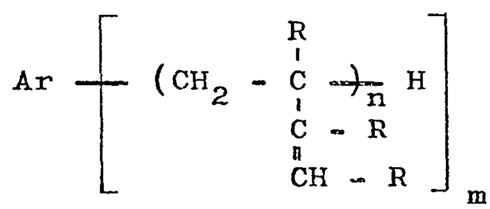

führt in denen Ar einen Arylrest darstellt, die Reste R unabhängig voneinander Wasserstoff oder Alkylreste und n bzw. m gleich 1 oder 2 sein können.

Solche Produkte fanden bisher nur relativ geringes Interesse und besaßen häufig den Charakter unerwünschter Nebenprodukte.

Sie besitzen durch ihren hohen Anteil an trans-Doppelbindungen schlechte lufttrocknende Eigenschaften und sind außerdem relativ leicht flüchtig. Längerkettige Telomere (n > 2) entstehen dabei normalerweise nur in geringem Umfang.

Produkte mit längeren Seitenketten können zwar nach den Verfahren der DE-PS 11 37 727 und der DE-PS 11 70 932 gewonnen werden, jedoch enthalten auch sie überwiegend trans-Doppelbindungen und immer noch einen beträchtlichen Anteil unerwünschter leicht flüchtiger Monoalkenylierungsprodukte

(vgl. auch H. Weber und B. Schleimer, Brennstoff-Chemie 49, 329 ff (1968)). Sie werden für Verwendungen auf dem Gebiet der Modifizierung von elastomeren und thermoplastischen Kunststoffen, der Schmiermittel und - nach Hydrierung - der Waschrohstoffe vorgeschlagen.

Bei Verwendung von Katalysatoren aus Cr(III)-Halogeniden und Alkylaluminiumhalogeniden gemäß dem Verfahren der US-PS 3 373 216 entstehen zwar Produkte, deren trans-Gehalt bereits geringer (35 bis 80 %) ist. Sie enthalten jedoch nur geringe Anteile eingebauter Aromaten.

In der japanischen Offenlegungsschrift 49-32985 wird ein Verfahren zur Herstellung von Polymeren, die aromatische Kohlenwasserstoffe an einer Polydienkette (der Einbau findet wahrscheinlich statistisch entlang der Polydienkette statt) enthalten, mit Hilfe eines durch Halogenverbindungen des Norbornens modifizierten Nickelkatalysators beschrieben. Diese Verfahrensweise führt zu Produkten, die z. B. auf 1 Mol Dien 0,5 Mol Aromaten und gegebenenfalls mehr als 70 % cis-1,4-Doppelbindungen enthalten.

Ein Verfahren, das ebenfalls zu Reaktionsprodukten aus konjugierten Diolefinen und aromatischen Kohlenwasserstoffen mit einem hohen prozentualen Anteil an cis-1,4-Doppelbindungen und - wie aus den Beispielen hervorgeht - einem nur geringen Anteil ($\leq$ 1 %) an 1,2-Doppelbindungen führt, ist Gegenstand der deutschen Patentanmeldung P 28 48 804.2. Das nicht zum Stand der Technik gehörende Verfahren ist dadurch gekennzeichnet, daß man konjugierte Diolefine in einem aromatischen Kohlenwasserstoff mit Hilfe eines Katalysators, bestehend aus einer in dem aromatischen Kohlenwasserstoff löslichen Nickelverbindung, einer halogenhaltigen aluminiumorganischen Verbindung, einem ausgewählten Modifikator und gegebenenfalls Wasser, polymerisiert.

Aufgabe der vorliegenden Erfindung war es, ein Verfahren

zu entwickeln, das es gestattet, in einfacher Weise Reaktionsprodukte aus konjugierten Diolefinen und aromatischen Kohlenwasserstoffen mit mehr als 1 % 1,2- und weniger als 35 % trans-1,4-Doppelbindungen in der Polymerkette herzustellen.

Diese Aufgabe wurde überraschenderweise durch die in den Ansprüchen beschriebene Maßnahme gelöst.

Überraschend deswegen, weil unter den Reaktionsbedingungen normalerweise eine Umsetzung zwischen der zur Modifizierung der Kobaltverbindung eingesetzten dreiwertigen organischen Phosphorverbindung und dem Modifikator stattfinden sollte, d. h. eine Inhibierung, zumindest aber eine Beeinträchtigung des Reaktionsverlaufs zu erwarten war.

Als konjugierte Diolefine können bei dem erfindungsgemäßen Verfahren alle 1,3-Diene des Standes der Technik (s. G. A. Olah, Friedel-Crafts and Related Reactions, Vol. II/1), wie z. B. Butadien, Isopren, Piperylen und 2,3-Dimethylbutadien eingesetzt werden, wobei Butadien bevorzugt ist.

Mit den konjugierten Dienen zur Reaktion gebracht werden können alle aromatischen Kohlenwasserstoffe, die unter den Reaktionsbedingungen keine oder nur geringe unerwünschten Nebenreaktionen verursachen, wie z. B. Benzol, Toluol, Xylole, Chlorbenzol, Cumol oder Alkenylaromaten, wie Styrol und Butenylbenzol. Selbstverständlich können auch Mischungen eingesetzt werden, ebenfalls solche, die neben dem (den) reaktiven aromatischen Kohlenwasserstoff(en) solche Kohlenwasserstoffe enthalten, die unter den angewandten Bedingungen nicht mit dem Dien reagieren, wie z. B. Hexan, Heptan, Octan und Cyclohexan.

Im allgemeinen wird das konjugierte Dien in einer Konzentration von 1 bis 50 Gewichtsprozent, bezogen auf den aromatischen Kohlenwasserstoff, eingesetzt. Der Konzentrationsbereich von 5 bis 40 Gewichtsprozent ist bevorzugt.

Geeignete Kobaltverbindungen zur Herstellung der Katalysatorkomponente 1 sind Salze organischer und anorganischer Säuren sowie Komplexverbindungen des Kobalts, z. B. Kobaltchlorid, Kobaltbromid, Kobaltjodid, Kobaltsulfat, Kobaltnitrat, Kobaltcarbonat, Kobaltphosphat, Kobaltsulfid, Kobaltcyanid, Kobaltcyanat, Kobalthydroxid, Kobaltacetat, Kobaltoxalat, Kobaltoctoat, Kobaltnaphthenat, Kobaltstearat, Kobaltpalmitat, Kobalt-bisacetylacetonat, Kobalt-bisacetoacetat, Dicyclopentadienylkobalt und Dikobaltoctacarbonyl. Bevorzugt sind Kobaltoctoat und Kobalt-bisacetylacetonat.

Die Löslichkeit der Kobaltverbindung in dem (den) aromatischen Kohlenwasserstoff(en) sollte vor oder nach der Behandlung mit einer dreiwertigen organischen Phosphorverbindung der allgemeinen Formel

$$P \begin{matrix} \diagup R^{17} \\ - R^{18} \\ \diagdown R^{19} \end{matrix} \qquad IV,$$

in der $R^{17}$, $R^{18}$ und $R^{19}$ unabhängig voneinander eine Alkyl-, Alkenyl- oder eine Arylgruppe oder eine allgemeine Gruppe $OR^{20}$, $OR^{21}$ und $OR^{22}$ sein können, wobei $R^{20}$, $R^{21}$ und $R^{22}$ wiederum unabhängig voneinander eine Alkyl-, Alkenyl- oder Arylgruppe sein können, 0,01 bis 1 g Co/l des aromatischen Kohlenwasserstoffs betragen.

Für die Modifizierung der Kobaltverbindungen geeignete dreiwertige organische Phosphorverbindungen sind einmal Alkyl-, Alkenyl- und Arylphosphine, und zum anderen Phosphite mit Alkyl-, Alkenyl- und/oder Arylgruppen. Sowohl in den Phosphinen wie in den Phosphiten können die Kohlenwasserstoffreste, die im allgemeinen bis zu 14, vorzugsweise bis zu 10 Kohlenstoffatome enthalten, gleich oder verschieden sein. Typische geradkettige, verzweigte oder cyclische gesättigte oder ungesättigte aliphatische Reste sind z. B. der Methyl-, Ethyl-, n-Propyl-, Isopropyl-, n-Butyl-, Isobutyl-, Hexyl-, Octyl-, Cyclohexyl-, Vinyl-, Allyl- und Crotylrest. Typische aromatische Reste sind der Phenyl-, Toluyl- und der Benzylrest.

Aus der Gruppe der Phosphine sind Triethyl-, Tri-n-butyl-, Triallyl- und Triphenylphosphin, aus der Gruppe der Phosphite Trimethyl-, Triethyl-, Tripropyl-, Tributyl-, Tricyclohexyl-, Triallyl-, Triphenyl-, Diphenylethyl-, Diphenylallyl-, Diphenylbutyl-, Diethylphenyl- und Dibutylphenylphosphit als Vertreter zu nennen. Bevorzugt werden bei dem erfindungsgemäßen Verfahren Triphenylphosphin, Tri-n-butylphosphin und Triphenylphosphit verwendet.

Als halogenhaltige aluminiumorganische Verbindung kommen solche der allgemeinen Formel $R_nAlX_{3-n}$, in der X gleich Fluor, Chlor, Brom oder Jod, R ein gegebenenfalls substituierter aliphatischer oder aromatischer Kohlenwasserstoffrest mit bis zu 14 Kohlenstoffatomen und n eine ganze oder gebrochene Zahl von 1 bis 2 sein kann, infrage. Bevorzugt stellt R einen aliphatischen Kohlenwasserstoffrest mit 1 bis 12, besonders bevorzugt mit 1 bis 4 Kohlenstoffatomen dar. Sowohl die Reste R als auch die Reste X können gleich oder verschieden sein.

Verbindungen, die innerhalb des für n angegebenen Bereiches allein oder im Gemisch eingesetzt werden können, sind solche der allgemeinen Formeln $RAlX_2$, $R_{1,5}AlX_{1,5}$ und $R_2AlX$, wobei die Methyl- und Ethylaluminiumhalogenide bevorzugt sind. Besonders bevorzugt sind bei dem erfindungsgemäßen Verfahren Diethylaluminiumchlorid ($Et_2AlCl$), Ethylaluminiumdichlorid ($EtAlCl_2$) und Ethylaluminiumsesquichlorid ($Et_{1,5}AlCl_{1,5}$).

Typische Vertreter der bei erfindungsgemäßen Verfahren einsetzbaren Modifikatoren gemäß Formel I sind tert.-Alkylchloride, -bromide und -jodide, wie z. B. tert.-Butyl- und Amylchlorid, -bromid und -jodid. Besonders geeignet sind tert.-Butylchlorid und -bromid sowie tert.-

Amylchlorid. Bevorzugte Vertreter für den Fall, daß $R^1$ ein Arylrest ist und die Reste $R^2$ und $R^3$ Wasserstoff darstellen, sind Benzylchlorid und Benzylbromid.

Typische Vertreter von Modifikatoren der allgemeinen Formel II sind z. B. Allyl-, Methallyl- und Crotylchlorid, -bromid und -jodid, wobei Allylchlorid, Allylbromid, Methallylchlorid und Crotylchlorid besonders bevorzugt sind. Ob es sich dabei um die cis- oder trans-Form oder um ein Isomerengemisch handelt, ist für das erfindungsgemäße Verfahren nicht kritisch.

Von den Modifikatoren der allgemeinen Formel III, in der die nicht zeichnerisch dargestellten Valenzen stets durch ein Wasserstoffatom abgesättigt sind, sind schließlich als typische Vertreter zu nennen: 2-Chlor-, -Brom- und -Jodnorbornan, 5-Chlor-, -Brom- und -Jodnorbornen-(2), Chlor-, Brom- und Jod-tricyclo-$[5,2,1,0^{2,6}]$-decan, 8(9)-Chlor-, -Brom- und -Jod-8,9-dihydro-tricyclo-$[5,2,1,0^{2,6}]$-dec-3-en. Besonders geeignet sind 2-Chlornorbornan und 8(9)-Chlor-8,9-dihydro-tricyclo-$[5,2,1,0^{2,6}]$-dec-3-en.

Neben den genannten Bestandteilen kann dem Katalysator gegebenenfalls als Katalysatorkomponente 4 eine H-acide Verbindung, wie z. B. Wasser, Alkohole oder organische Säuren, zugesetzt werden.

Die Kobaltverbindung wird im allgemeinen in Mengen von 0,01 bis 1 Mol, bezogen auf 1 Mol der halogenhaltigen aluminiumorganischen Verbindung (2) eingesetzt. Bevorzugt sind 0,02 bis 0,5 Mol, bezogen auf 1 Mol (2).

Der Anteil der zur Modifizierung der Kobaltverbindung eingesetzten dreiwertigen organischen Phosphorverbindung beträgt im allgemeinen 0,1 bis 50 Mol, vorzugsweise 0,3 bis 10 Mol, bezogen auf 1 Mol Kobaltverbindung.

Die halogenhaltige aluminiumorganische Verbindung (2) wird im allgemeinen in einer Menge von 0,0005 bis 0,02 Mol, bezogen auf 1 Mol des konjugierten Diens, eingesetzt. Bevorzugt ist eine Menge von 0,001 bis 0,01 Mol.

Schließlich wird der Modifikator (3) im allgemeinen in einer Menge von 0,01 bis 20 Mol, vorzugsweise 0,1 bis 5 Mol, bezogen auf 1 Mol der halogenhaltigen aluminiumorganischen Verbindung, eingesetzt.

Sofern dem Katalysator noch eine H-acide Verbindung zugesetzt wird, geschieht dies in einer Menge von 0,05 bis 10 Mol, vorzugsweise 0,1 bis 1 Mol, bezogen auf 1 Mol der halogenhaltigen aluminiumorganischen Verbindung.

Das erfindungsgemäße Verfahren wird vorteilhafterweise in einer Apparatur durchgeführt, in der gefahrlos ein stärkerer Temperatur- und Druckanstieg beherrscht werden kann, d. h. in die auch noch bei steigendem Druck die kontinuierliche oder portionsweise Zugabe des Diens möglich ist. Diese Bedingungen werden z. B. von einem Autoklaven erfüllt. Die Umsetzung kann jedoch auch in batch-Fahrweise mit einmaligem Dienzusatz, z. B. im Reaktionskolben oder in einem Druckrohr, durchgeführt werden.

Die Reaktionstemperatur kann im weiten Bereich variiert werden, z. B. von -50 bis +200 °C, bevorzugt ist jedoch der Temperaturbereich von -20 bis +130 °C und besonders bevorzugt der Bereich von -10 bis +100 °C. Der Reaktionsdruck beträgt im allgemeinen bis zu 30 bar, die Reaktionszeit 0,1 bis 5 Stunden.

Aufarbeitung und Reinigung der nach dem beanspruchten Verfahren hergestellten Reaktionsprodukte gestalten sich z. B. derart, daß zunächst der Katalysator mit der notwendigen Menge von z. B. Wasser, Alkohol oder Aceton desaktiviert und anschließend der Reaktionsansatz zunächst mit z. B. Bleicherde und gegebenenfalls einer basischen Verbindung, wie Natriumcarbonat, Kaliumcarbonat oder Calciumoxid verrührt und filtriert wird. Wurden als organische Phosphorverbindungen Ester der phosphorigen Säure eingesetzt, die aus dem Reaktionsprodukt entfernt werden sollen, kann dies durch eine Behandlung mit Heißdampf oder heißem Wasser erfolgen.

Die Menge der zugesetzten Bleicherde wird durch die Katalysatorkonzentration und die Ansatzgröße bestimmt. Ein ausreichender Zusatz kann durch orientierende Versuche leicht ermittelt werden.

Dann wird das überschüssige Lösemittel - gegebenenfalls nach Zusatz eines Stabilisators, wie z. B. 2,6-Di-tert.-butyl-p-kresol, abdestilliert. Für diesen Aufarbeitungsschritt sind nicht nur die klassischen konventionellen Methoden brauchbar, sondern z. B. auch das Abdestillieren am Dünnschichtverdampfer. Dabei können auch, falls es gewünscht wird, niedrigsiedende Reaktionsprodukte mit abdestilliert werden. Es ist besonders bei höhermolekularen Produkten auch eine Isolierung der Reaktionsprodukte durch Zusatz eine Fällungsmittels, das eine Phasentrennung bewirkt, möglich. Hierbei kann eine Fraktionierung stattfinden. Geeignete Fällmittel sind z. B. Wasser und/oder Methanol, Ethanol, Aceton.

Auf dem beschriebenen Weg können Produkte mit einem breiten Eigenschaftsbild erhalten werden, z. B. solche mit Viskositäten von 30 bis $10^5$ mPa•s, aber auch feste Polymere, und Jodzahlen zwischen 50 und dem Bereich der reinen Polydiolefine.

Der Anteil an 1,2- oder Vinyldoppelbindungen in den nach dem erfindungsgemäßen Verfahren hergestellten Reaktionsprodukten kann im weiten Rahmen variiert werden. Er beträgt stets mehr als 1 % und liegt im allgemeinen zwischen 5 und 30 %. Die restlichen Doppelbindungen sollen überwiegend cis-1,4-Konfiguration und maximal 35 % trans-1,4-Konfiguration besitzen.

Eine Steuerung des Arylgruppeneinbaus ist durch die Anpassung der Reaktionsbedingungen möglich und wird am einfachsten spektroskopisch durch das Vorliegen substituierter Aromaten und den Anteil aromatischer Protonen bestimmt. Letzterer liegt durchschnittlich zwischen 3 bis 25 %, bezogen auf den Gesamtanteil an Protonen.

Die nach dem erfindungsgemäßen Verfahren erhaltenen Reaktionsprodukte eignen sich besonders - gegebenenfalls nach vorangegangener Modifizierung, wie z. B. Maleinsäureanhydridanlagerung, Halogenierung, Hydrierung usw. - zur Herstellung umweltfreundlicher, lufttrocknender Überzugsmittel.

Das erfindungsgemäße Verfahren wird durch die nachfolgenden Beispiele näher erläutert.

Die darin angegebenen Kenngrößen wurden wie folgt bestimmt:

1. Jodzahl: M. E. Tunnicliffe et al., European Polym. J. (1965) 1, 260,
2. NMR (Mikrostruktur und Protonenverteilung): E. Pretsch et al., Tabellen zur Strukturaufklärung org. Verbindungen, Springer Verlag, 1976,
3. IR: P. Simák und G. Fahrbach, Angew. Makromol. Chem. (1970) 12, 73-88,
4. Raman: B. Schrader, Angewandte Chemie (1973) 85, 925.

Beispiel 1

In einem 2,5 l-Glasautoklaven mit Doppelmantel und Seitentubus wurden 1,7 l über Molekularsieb getrocknetes Toluol von 30 °C vorgelegt und 125 g trocknes Butadien zugesetzt.

Anschließend erfolgte nacheinander der Zusatz von 0,75 mMol Kobaltoctoat (als 0,1 molare Lösung in Toluol), 1,5 mMol Triphenylphosphit (1 molar in Toluol), 20 mMol Diethylaluminiumchlorid (1 molar in Toluol) und 11 mMol Wasser. Nach Anstieg der Innentemperatur um 5 ° wurden 8 mMol 5-Chlor-2-norbornen zugesetzt und rasch weitere 125 g Butadien eingeleitet. Die Temperatur stieg hierbei auf 59 °C.

Nach einer Reaktionszeit von 2 Stunden bei 50 °C wurde die toluolische Lösung abgelassen, mit Methanol desaktiviert, ca. 20 g Natriumcarbonat-Monohydrat und 30 g Bleicherde zugesetzt und filtriert. Nach dem Abdestillieren des Lösungsmittels Toluol bei 120 °C im Vakuum hinterblieben 223 g eines hellgelben Öls mit der Viskosität 500 mPa·s und einer Jodzahl von 268. Der IR-spektroskopisch bestimmte Doppelbindungsgehalt betrug 12 % 1,2-, 24 % trans-1,4- und 64 % cis-1,4-Einheiten. Aus dem $^1$H-NMR-Spektrum errechneten sich 12,4 % aromatische und 19,7 % olefinische Protonen.

Beispiel 2

Es wurde wie in Beispiel 1 verfahren, jedoch erfolgt die Reagenzzugabe bei 50 °C in der Reihenfolge 1,7 l trocknes Toluol, 11 mMol Wasser, 125 g Butadien, 20 mMol $(C_2H_5)_2AlCl$, 8 mMol 2-Methylallylchlorid, 1,5 mMol Triphenylphosphit, 0,75 mMol Kobaltoctoat und danach weitere 125 g Butadien.

Die Innentemperatur stieg auf 84 °C. Nach der Aufarbeitung wurden 233 g eines Öls mit der Viskosität 2 100 mPa·s und der Jodzahl 227. Der Doppelbindungsgehalt betrug 5 % 1,2-, 26 % trans-1,4- und 69 % cis-1,4-Einheiten.

Aus dem $^1$H-NMR-Spektrum ergaben sich 16 % aromatische und 15,3 % olefinische Protonen.

Vergleichsbeispiel A

250 g Butadien wurden in eine Lösung des präformierten Katalysators, bestehend aus 1 mMol Kobaltoctoat, 5 mMol Tri-n-butylphosphin und 10 mMol $Et_3Al_2Cl_3$ - gelöst in 2 l trocknem Toluol von 50 °C -, eingeleitet. Die Reaktionstemperatur stieg auf maximal 80 °C; die Reaktionszeit betrug 2 Stunden.

Nach der in Beispiel 1 beschriebenen Aufarbeitung wurden 110 g hochviskoses Polybutadien mit einer Jodzahl von 454 erhalten. Die Viskosität lag bei 2 500 mPa·s. IR-spektroskopisch wurden 35 % 1,2-, 8 % trans-1,4- und 57 % cis-1,4-Doppelbindungen nachgewiesen. Im $^1$H-NMR-Spektrum waren nur 0,4 % aromatische Protonen neben 39,4 % olefinischen und 60,2 % aliphatischen Protonen zu erkennen. Der Resttoluolgehalt lag bei 0,33 %.

Das Beispiel zeigt, daß ohne Einsatz eines erfindungsgemäßen Chlorkohlenwasserstoffs als Modifikator kein nennenswerter Einbau von Arylgruppen erfolgt.

Vergleichsbeispiel B

Es wurde wie im Vergleichsbeispiel A gearbeitet, jedoch 10 mMol 1-Chlorbutan nach der Katalysatorpräformierung zugefügt. Die Innentemperatur stieg während der Polymerisation von 50 °C auf maximal 82 °C. Es wurden 95 g eines hochviskosen Polybutadienöls mit der Viskosität $10^4$ mPa·s und einer Jodzahl von 446 erhalten. Es besaß eine IR-spektroskopisch bestimmte Mikrostruktur von 35 % 1,2-, 10 % trans-1,4- und 55 % cis-1,4-Doppelbindunge Im $^1$H-NMR-Spektrum wurden - bei einem Resttoluolgehalt < 1 % - weniger als 0,5 % aromatische Protonen nachgewiesen.

Das Beispiel zeigt, daß beim Einsatz eines Halogenkohlenwasserstoffs, der nicht unter die allgemeinen Formeln I bis III fällt, kein nennenswerter Einbau von Arylgruppen

erreicht wird.

<u>Beispiele 3 bis 9</u>

Es wurde entsprechend Beispiel 1 verfahren. Soweit Änderungen erfolgten, sind diese in der folgenden Tabelle - zusammen mit den Versuchsresultaten -, vermerkt. Als Kobaltverbindung wurde in allen Fällen Kobaltoctoat eingesetzt.

Tabelle

| Bsp. Nr. | Art und Menge (mMol) der Katalysatorkomponenten | | | Art und Menge (mMol) des Modifikators | Ausbeute (g) | Anfangstemperatur (°C) | maximale Temperatur (°C) | Reaktionszeit (min) | Viskosität (mPa·s) | Jodzahl | Mikrostruktur (%) | | |
|---|---|---|---|---|---|---|---|---|---|---|---|---|---|
| | Co-Verb. | P-Verbindung | Al-org. Verb. | | | | | | | | cis-1,4 | trans-1,4 | 1,2 |
| 3 | 0,75 | $P(OPh)_3$; 1,5 | $Et_3Al_2Cl_3$; 10 | A 1); 8,0 | 146 | 35 | 68 | 30 | 2 500 | 291 | 68 | 24 | 8 |
| 4 | 0,75 | $PPh_3$; 3,75 | $Et_3Al_2Cl_3$; 10 | 2-Methylallylchlorid 8,0 | 168 | 50 | 104 | 60 | 25 000 | 307 | 67 | 19 | 14 |
| 5 | 0,75 | $PPh_3$; 3,75 | $Et_3Al_2Cl_3$; 10 | 2-Methylallylchlorid 8,0 | 258 | 50 | 112 | 60 | 8 000 | 176 | n.a. 4) | 15 5) | 3 5) |
| 6 | 0,75 | $P(C_4H_9-n)_3$; 3,75 | $Et_3Al_2Cl_3$; 10 | A 1); 8,0 | 225 | 30 | 99 | 15 | 39 000 | 379 | 65 | 16 | 19 |
| 7 | 1,0 | $P(C_4H_9-n)_3$; 5,0 | $Et_2AlCl$ plus 11,1 mMol $H_2O$ 20 | A 1); 8,0 | 203 | 50 | 102 | 40 | 22 000 | 361 | 63 | 16 | 21 |
| 8 | 1,0 | $P(C_4H_9-n)_3$; 5,0 | $EtAlCl_2$; 20 | A 1); 8,0 | 245 | 30 | 110 | 30 | 28 000 | 240 | 67 | 25 | 8 |
| 9 | 1,0 | $P(OPh)_3$; 2,0 | $EtAlCl_2$; 20 | A 1); 10 | 380 | 45 | 140 | 30 | 230 | 186 | n.a. 4) | 17 5) | 2 5) |

Tabelle - Fortsetzung

| Bsp. Nr. | Resttoluol (Gew.-%) | Protonenverteilung (%) aromat.[2)] | olef. | benzyl | aliph.[3)] | Bemerkungen |
|---|---|---|---|---|---|---|
| 3 | < 0,01 | 9,7 | 20,8 | 5,0 | 64,5 | ohne $H_2O$ als Katalysatorkomponente |
| 4 | 5,2 | 5,0 | 22,1 | 2,0 | 67,7 | ohne $H_2O$ als Katalysatorkomponente |
| 5 | < 0,1 | 15,2 | 9,6 | 3,9 | 70,1 | ohne $H_2O$ als Katalysatorkomponente |
| 6 | < 0,05 | 3,9 | 29,6 | 1,6 | 64,9 | ohne $H_2O$ als Katalysatorkomponente |
| 7 | 0,76 | 4,6 | 28,9 | 2,7 | 63,3 | Gesamtmenge Butadien vorgelegt, Zusatz von 10 mMol $Et_3Al_2Cl_3$ 1 min nach der Butadiendosierung |
| 8 | 0,51 | 11,8 | 15,7 | 4,7 | 67,5 | Gesamtmenge Butadien vorgelegt, ohne $H_2O$ als Katalysatorkomponente |
| 9 | - | - | - | - | - | Gesamtmenge Butadien vorgelegt, ohne $H_2O$ als Katalysatorkomponente |

1) A = 8(9) Chlor-8,9-dihydrodicyclopentadien
2) unter Berücksichtigung des Resttoluolgehalts
3) einschließlich der Methylprotonen des Toluols
4) nicht auswertbar
5) Absolutwerte

Patentansprüche:

1. Verfahren zur Herstellung von Reaktionsprodukten aus konjugierten Diolefinen und aromatischen Kohlenwasserstoffen mit Hilfe eines Katalysators bestehend aus

   1 einer in dem aromatischen Kohlenwasserstoff löslichen Verbindung eines Übergangselementes der 8. Gruppe des Periodischen Systems der Elemente,

   2 einer halogenhaltigen aluminiumorganischen Verbindung und

   3 einem Modifikator,

   dadurch gekennzeichnet,
   daß man als Katalysatorkomponente 1 eine phosphormodifizierte Kobaltverbindung und als Katalysatorkomponente 3 eine Verbindung der allgemeinen Formeln

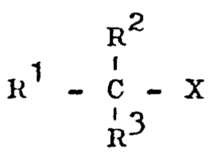

I,

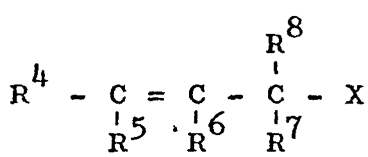

II oder

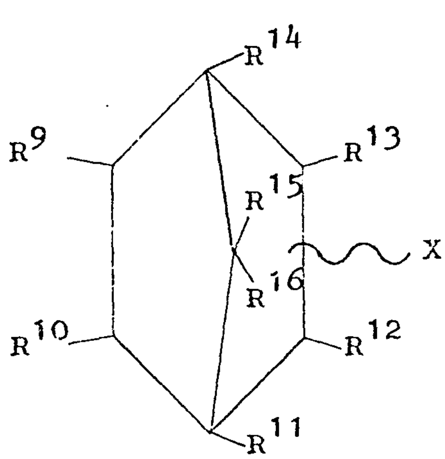

III

verwendet, wobei

3.1 X jeweils Chlor, Brom oder Jod und

3.2 $R^1$ bis $R^{16}$ unabhängig voneinander

3.2.1 gegebenenfalls durch Halogenatome substituierte geradkettige oder verzweigte gesättigte oder ungesättigte aliphatische oder cycloaliphatische Kohlenwasserstoffreste mit 1 bis 20 Kohlenstoffatomen oder

3.2.2 gegebenenfalls durch 1 bis 5 geradkettige oder verzweigte - gegebenenfalls durch Halogenatome substituierte - gesättigte oder ungesättigte aliphatische oder cycloaliphatische Kohlenwasserstoffreste mit 1 bis 10 Kohlenstoffatomen und/oder durch Halogenatome substituierte Arylreste mit 6 bis 14 Kohlenstoffatomen bedeuten,

3.2.3 $R^2$ bis $R^{16}$ auch Wasserstoff sein können, jedoch $R^2$ und $R^3$ nur dann, wenn $R^1$ einen Arylrest der unter 3.2.2 angegebenen Bedeutung darstellt, und

3.2.4 $R^9$ und $R^{10}$ zusammen auch Bestandteile eines anellierten - gegebenenfalls substituierten - Ringsystems sein können.

2. Verfahren nach Anspruch 1,
dadurch gekennzeichnet,
daß man als weitere Katalysatorkomponente eine H-acide Verbindung (4) einsetzt.

**0032167**

Nummer der Anmeldung

EP 80 10 6894

Europäisches Patentamt

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D/A | US - A - 3 373 216 (E. TORNQUIST)<br>* Patentanspruch 1; Spalte 1, Zeile 52 * | |
| A | DE - B - 1 196 186 (BASF)<br>* Patentanspruch 1; Beispiel 70 * | |
| A | FR - A - 2 254 587 (HULS)<br>* Patentanspruch 1 *<br>& DE - A - 2 361 782 | |
| P/A/D | DE - A - 2 848 804 (HULS)<br>* Patentanspruch * | |

KLASSIFIKATION DER ANMELDUNG (Int. Cl.)

C 07 C 15/44
C 08 F 2/38
4/70
36/04

RECHERCHIERTE SACHGEBIETE (Int. Cl.)

C 07 C 2/72
15/40
C 08 F 2/38
4/70
36/04
136/04
236/04

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsatze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentanspruche erstellt.

| Recherchenort | Abschlußdatum der Recherche | Prüfer |
|---|---|---|
| Den Haag | 17-03-1981 | V. HUMBEECK |